# EUROPÄISCHE PATENTSCHRIFT

(11) **EP 3 678 606 B1**
(45) Veröffentlichungstag und Bekanntmachung des Hinweises auf die Patenterteilung: **10.01.2024**
(21) Anmeldenummer: 18746947.3
(22) Anmeldetag: 30.07.2018
(51) Int. Cl.: A61F 2/54, A61F 2/60, A61F 2/68, A61F 2/70, A61F 5/01, F16D 7/00, F16D 41/066, H02K 49/10

(54) **ORTHOPÄDIETECHNISCHE EINRICHTUNG MIT ÜBERLASTSCHUTZ**
ORTHOPEDIC DEVICE WITH OVEROAD PROTECTION
DISPOSITIV ORTHOPÉDIQUE AVEC PROTECTION CONTRE LES SURCHARGE

(30) Priorität: 06.09.2017 DE 102017120463
(43) Veröffentlichungstag der Anmeldung: 15.07.2020
(73) Patentinhaber: Otto Bock Healthcare Products GmbH, 1110 Wien (AT)
(72) Erfinder: SAGMEISTER, Luis, 2823 Pitten (AT)
(74) Vertreter: Gramm, Lins & Partner Patent- und Rechtsanwälte PartGmbB
(86) Internationale Anmeldenummer: PCT/EP2018/070604
(87) Internationale Veröffentlichungsnummer: WO 2019/048136

(56) Entgegenhaltungen:
- DE-A1-102012 023 173
- US-A- 3 240 304
- US-A- 4 651 856
- US-A1- 2015 216 679

## Beschreibung

Die Erfindung betrifft eine orthopädietechnische Einrichtung mit einem Endeffektor und einer Antriebseinrichtung, die eingerichtet ist, den Endeffektor durch Übertragen eines Drehmomentes von der Antriebseinrichtung auf den Endeffektor anzutreiben.

Derartige orthopädietechnische Einrichtungen können beispielsweise Prothesen, insbesondere Handprothesen sein, wobei der Endeffektor in diesem Fall beispielsweise durch einen oder mehrere Finger gebildet wird. Selbstverständlich sind auch andere orthopädietechnische Einrichtungen, beispielsweise Orthesen oder andere Prothesen denkbar, bei denen der Endeffektor auf andere Weise ausgebildet ist.

In vielen Ausgestaltungen einer derartigen orthopädietechnischen Einrichtung ist es von Vorteil, dass eine einmal erreichte Position des Endeffektors, beispielsweise eine Position eines Fingers einer Prothesenhand, durch äußere Einflüsse, also insbesondere ein auf den Endeffektor von außen wirkendes Drehmoment, nicht oder nicht sofort geändert werden kann. Greift der Benutzer einer derartigen orthopädietechnischen Einrichtung beispielsweise mit einer Prothesenhand einen Gegenstand, sollte dieser Gegenstand nicht fallen gelassen werden. Bei Prothesen der oberen Extremität wird beispielsweise das Greifen, Heben oder Drehen von Endeffektoren meist mittels Elektromotoren und entsprechenden Getrieben realisiert. In den meisten dieser Fälle soll die einmal aufgebrachte Kraft, beispielsweise eine Griffkraft, auch nach dem Ausschalten des Stroms des Elektromotors aufrechterhalten bleiben. Dies soll jedoch erreicht werden, ohne dass es notwendig ist, den Strom aufrechtzuerhalten, um Energie zu sparen. Üblicherweise wird diese Aufgabe durch ein Gesperre gelöst, welches den Antriebsstrang gegen Betätigung durch externe Kräfte blockiert. Durch Manipulieren von gegriffenen Gegenständen wie etwa Heben können deutlich höhere Kräfte von außen auf das Gesperre wirken welche zu einer Beschädigung des Gesperres führen können. In diesem Fall muss das Gesperre geschützt werden.

Dies wird mit der vorliegenden Erfindung erreicht. Wird das von außen wirkende Drehmoment jedoch so groß, dass Bauteile, etwa das Getriebe, die Antriebseinrichtung oder ein Gesperre, beschädigt werden könnten, muss der Endeffektor bewegt werden, um eine Beschädigung zu verhindern. Ein beschädigtes Gesperre könnte beispielsweise dazu führen, dass ein Notöffnen des Effektors, das für einen Notfall vorgesehen sein kann, nicht mehr ausgeführt werden könnte.

Derart von außen, also nicht durch die Antriebseinrichtung, erzeugte, auf den Endeffektor wirkenden Kräfte und/oder Drehmomente können jedoch so groß sein, dass sie zu einer Beschädigung oder Zerstörung des Endeffektors und damit zu einer starken Beschädigung der orthopädietechnischen Einrichtung führen können. Dies ist natürlich zu vermeiden, da die orthopädietechnische Einrichtung funktionsfähig gehalten werden soll und zudem die andernfalls anfallenden Reparaturkosten zu vermeiden sind.

Aus der DE 10 2012 023173 A1 ist beispielsweise eine Prothese bekannt, die ein Kupplung, insbesondere eine Rutschkupplung aufweist. Bei Überschreiten eines Schwellendrehmoments reduziert oder verhindert die Kupplung eine weitere Drehmomentübertragung. Bis zu der Schwelle gestattet sie sie jedoch.

Die US 3 240 301 A beschreibt eine Kupplung, die bei Überschreiten eines Grenzdrehmoments die Drehmomentübertragung zwischen einem Antrieb und einem Abtrieb trennt. Zusätzlich wird die Kupplung durch einen Magneten in der entkoppelten Position gehalten.

Der Erfindung liegt daher die Aufgabe zugrunde, eine orthopädietechnische Einrichtung der eingangs erwähnten Art so weiterzuentwickeln, dass eine einmal erreichte Position und/oder Lage des Endeffektors erreicht und gehalten werden kann und dennoch eine Beschädigung des Endeffektors sicher vermieden wird.

Die Erfindung löst die gestellte Aufgabe durch eine orthopädietechnische Einrichtung gemäß dem Oberbegriff des Anspruchs 1, die sich dadurch auszeichnet, dass sie einen Überlastschutz aufweist, der eingerichtet ist, eine Übertragung eines Drehmomentes von dem Endeffektor auf die Antriebseinrichtung nur bis zu einem Grenzdrehmoment in zumindest einer Richtung zu verhindern, wobei der Überlastschutz wenigstens ein erstes Kopplungselement und ein zweites Kopplungselement aufweist, die relativ zu einander drehbar und magnetisch gekoppelt sind.

Durch einen derartigen Überlastschutz werden von außen wirkende Drehmomente an die vorzugsweise unbestromte Antriebseinrichtung derart weitergeleitet, dass der Endeffektor zumindest in einer Richtung durch die von außen wirkenden Drehmomente bewegbar ist, sodass das Gesperre keinen Schaden nimmt. Dabei kann es durchaus ausreichend sein, diese Bewegung durch von außen angreifende Drehmomente nur in einer Richtung zu erlauben. So ist beispielsweise bei einer geschlossenen Prothesenhand ein Drehmoment, das in Richtung auf ein weiteres Schließen der Prothesenhand gerichtet ist, unproblematisch. Lediglich ein in die entgegengesetzte Richtung wirkendes Drehmoment, das folglich versucht, die Hand zu öffnen, darf nicht in eine derartige Bewegung umgesetzt werden, solange es das Gesperre nicht beschädigen kann. In anderen Situationen, beispielsweise wenn eine geöffnete Prothesenhand auf einen zu greifenden Gegenstand zubewegt wird, ist ein Drehmoment, das in Richtung eines weiteren Öffnens der Hand gerichtet ist, unproblematisch, während ein von außen wirkendes Drehmoment, das darauf gerichtet ist, die Hand zu schließen, etwa weil der Träger der Prothesenhand mit der Hand gegen einen Gegenstand, beispielsweise gegen eine Wand, anstößt, nicht zu einer entsprechenden Bewegung führen darf, es sei denn, das Gesperre könnte beschädigt werden.

Durch einen derartigen Überlastschutz wird neben der Antriebseinrichtung und dem Getriebe insbesondere auch das Gesperre, das eine Weiterleitung des Drehmomentes bis zu dem Grenzdrehmoment verhindert und in einer bevorzugten Ausgestaltung Teil des Überlastschutzes ist, geschützt. Das von außen auf den Endeffektor wirkende Drehmoment wird von dem Gesperre aufgenommen. Überschreitet es das Grenzdrehmoment wird es entkoppelt und nicht mehr von dem Gesperre aufgenommen. So wird verhindert, dass das Gesperre beschädigt werden kann.

Vorteilhafterweise verhindert der Überlastschutz eine Übertragung des Drehmomentes von dem Endeffektor auf die Antriebseinrichtung bis zu einem Grenzdrehmoment in beiden Richtungen. Dies bedeutet, dass ein Drehmoment, das kleiner als das Grenzdrehmoment ist und somit das Gesperre oder ein anderes Bauteil nicht beschädigen kann, abgefangen wird. Ein Drehmoment, das größer als das Grenzdrehmoments ist, könnte das Gesperre oder ein anderes Bauteil beschädigen. Daher führt der Überlastschutz zu einer Bewegung des Endeffektors um das Drehmoment abzubauen und die Bauteile vor Beschädigungen zu schützen.

Erfindungsgemäß verfügt der Überlastschutz über wenigstens ein erstes Kopplungselement und ein zweites Kopplungselement, die relativ zueinander drehbar und magnetisch gekoppelt sind.

Die Kopplungselemente sind vorzugsweise so angeordnet, dass ein Drehmoment, das auf den Endeffektor wirkt, auf das wenigstens eine erste Kopplungselement oder das zweite Kopplungselement übertragen wird. Die beiden Kopplungselemente sind magnetisch gekoppelt, sodass eine Kraft überwunden werden muss, um die beiden Kopplungselemente relativ zueinander zu drehen. Dazu ist ein Drehmoment notwendig. Sofern das von außen auf den Endeffektor wirkende Drehmoment diesen Grenzdrehmomentwert nicht überschreitet, ist die magnetische Kopplung zwischen den Kopplungselementen so stark, dass es nicht zu einer Rotation und damit nicht zu einem Bewegen des Endeffektors kommt. Erst wenn dieses Grenzdrehmoment überschritten wird, wird die durch die magnetische Kopplung aufgebrachte Kraft überwunden und es kommt zu einer Bewegung der Kopplungselemente relativ zueinander. Dadurch wird gewährleistet, dass ein auf den Endeffektor wirkendes Drehmoment, das größer ist als das Grenzdrehmoment, keine Bauteile beschädigen kann.

Dadurch, dass eine magnetische Kopplung vorgesehen ist, kann der Überlastschutz räumlich klein ausgebildet werden, was insbesondere bei Prothesenhänden, aber auch bei vielen anderen orthopädietechnischen Einrichtungen von Vorteil ist. Zudem kann die Haltekraft, die durch die Magneten aufgebracht und von dem Drehmoment überwunden werden muss, berührungslos übertragen werden, sodass es nicht zu Reibungseffekten und insbesondere nicht zu dadurch hervorgerufenem Verschleiß kommt.

Vorteilhafterweise verfügt das zweite Kopplungselement über eine Mehrzahl von Magneten, vorzugsweise Permanentmagneten. Auf diese Weise wird die magnetische Haltekraft durch die Mehrzahl von Magneten aufgebracht, was eine homogenere Verteilung der Haltekraft beispielsweise in Umfangsrichtung ermöglicht und zudem erlaubt, den Überlastschutz und damit die orthopädietechnische Einrichtung insgesamt räumlich klein auszugestalten. In einer bevorzugten Ausgestaltung sind die Magneten lösbar in dafür an dem zweiten Kopplungselement vorhandenen Vertiefungen und/oder Ausnehmungen angeordnet. Dadurch wird gewährleistet, dass die durch die Magneten zwischen den Kopplungselementen herrschenden Kopplungskräfte individuell und an die jeweiligen Bedürfnisse angepasst werden können. Dieses ist besonders einfach möglich, indem Magneten aus den entsprechenden Vertiefungen und/oder Ausnehmungen herausgenommen werden, um die magnetischen Haltekräfte zu reduzieren oder indem zusätzliche Magneten in die Vertiefungen und/oder Ausnehmungen aufgenommen werden, um die magnetischen Haltekräfte zu vergrößern. Damit kann das Grenzdrehmoment eingestellt und insbesondere so gewählt werden, dass Drehmomente, die kleiner als das eingestellte Grenzdrehmoment Bauteile wie das Getriebe, das Gesperre oder die Antriebseinrichtung nicht beschädigen können.

Besonders bevorzugt sind die Vertiefungen und/oder Ausnehmungen äquidistant über zumindest einen Abschnitt des Umfanges, vorzugsweise den vollständigen Umfang, des zweiten Kopplungselementes angeordnet.

Es hat sich als vorteilhaft herausgestellt, dass die orthopädietechnische Einrichtung zwei erste Kopplungselemente aufweist, zwischen denen das zweite Kopplungselement angeordnet ist. Auf diese Weise können die von den Magneten erzeugten Kräfte auf beiden Seiten der Magneten, insbesondere an ihren beiden Pol-Enden genutzt werden. In einer bevorzugten Ausgestaltung weist wenigstens eines der Kopplungselemente ein magnetisierbares Material auf oder besteht daraus. Auf diese Weise lassen sich magnetische Feldlinien ins Material lenken, was eine besonders hohe magnetische Kraft zur Folge hat. Vorzugsweise sind alle ersten Kopplungselemente gleich ausgebildet.

In einer bevorzugten Ausgestaltung verfügt wenigstens eines der ersten Kopplungselemente, vorteilhafterweise jedes der ersten Kopplungselemente, über eine Mehrzahl von Kopplungsbereichen, die insbesondere in Form von radial nach innen ragenden Laschen, ausgebildet sind, wobei vorzugsweise eine Anzahl der Laschen der Anzahl der Magneten und/oder einer Anzahl der Vertiefungen und/oder Ausnehmungen entspricht. Die ersten Kopplungselemente sind in diesem Fall besonders bevorzugt in Form von Ringen oder ringförmigen Scheiben ausgebildet, die die Kopplungsbereiche aufweisen. Selbstverständlich sind auch andere Kopplungsbereiche, beispielsweise in Form von nach außen ragenden Laschen oder in Form von Elementen aus besonders gut magnetisierbarem Material, möglich. Insbesondere für den Fall, dass die Anzahl der Laschen der Anzahl der Vertiefungen und/oder Ausnehmungen entspricht, ist gewährleistet, dass unabhängig von der Anzahl der Magneten, die sich in den Vertiefungen und/oder Ausnehmungen befinden, immer ein möglichst guter magnetischer Kontakt zwischen den verschiedenen Kopplungselementen erreicht wird. Wird in einem solchen Fall durch ein extern angreifendes Drehmoment, das auf den Endeffektor wirkt, das Grenzdrehmoment überschritten, bewegt sich das zweite Kopplungselement relativ zu den ersten Kopplungselementen, sodass jeder der Magneten von dem bei ihm angeordneten Kopplungsbereichen wegbewegt wird und nach einer vorbestimmten Drehung dem nächsten Kopplungsbereich zugeordnet ist. Hier ist die durch die Magneten aufgebrachte Haltekraft erneut maximal, sodass eine weitere Drehung der Kopplungselemente relativ zueinander verhindert wird, sofern nicht das extern angreifende Drehmoment weiterhin das Grenzdrehmoment überschreitet.

In einer besonders bevorzugten Ausgestaltung ist radial außerhalb des zweiten Kopplungselementes, das bevorzugt ebenfalls als ringförmige Scheibe ausgebildet ist, ein zusätzlicher Laufring angeordnet, der drehfest mit den beispielsweise zwei ersten Kopplungselementen verbunden ist und auf diese Weise auch die beiden ersten Kopplungselemente miteinander verbindet. Da sich dieser Ring radial außerhalb des zweiten Kopplungselementes befindet, kann auf diese Weise ein geschlossener Magnetlinienkreis erreicht werden.

Bevorzugt ist, dass das wenigstens eine erste Kopplungselement oder das zweite Kopplungselement drehfest an einem Gehäuse angeordnet ist.

Vorteilhafterweise verfügt der Überlastschutz über eine Sperreinrichtung, die eingerichtet ist, die Übertragung des Drehmomentes von der Antriebseinrichtung auf den Endeffektor nicht zu beeinflussen und die Übertragung des Drehmomentes von dem Endeffektor auf die Antriebseinrichtung bis zu dem Grenzdrehmoment zu verhindern.

Durch eine solche Sperreinrichtung wird erreicht, dass eine Bewegung des Endeffektors durch die Antriebseinrichtung selbst bei kleinen und kleinsten Drehmomenten erfolgt, während Drehmomente von außen, die unabhängig von der Richtung auf den Endeffektor einwirken, nicht zu einer Bewegung des Endeffektors führen, sofern sie nicht das Grenzdrehmoment überschreiten.

Vorzugsweise ist die Sperreinrichtung ein Klemmrollengesperre mit einem Gehäuse und einer Mehrzahl von Klemmscheiben, die in dem Gehäuse drehbar angeordnet sind und jeweils wenigstens eine Ausnehmung, in der sich eine Klemmrolle befindet, wenigstens einen Antriebsvorsprung und wenigstens einen Abtriebsvorsprung aufweisen, wobei die Klemmscheiben derart angeordnet und ausgebildet sind, dass ein Drehen der Klemmscheiben durch Antreiben der Antriebsvorsprünge möglich ist und beim Antreiben der Abtriebsvorsprünge ein Drehen der Klemmscheiben verhindert wird. Über die Anzahl der verwendeten Ausnehmungen und Klemmrollen lässt sich die Sperrkraft nahezu beliebig skalieren. Neben einem Klemmrollengesperre können alle Arten von Gesperren geschützt werden, wie etwa auch das Schlingfedergesperre.

Bevorzugt sind die Ausnehmungen der Klemmscheiben nach radial außen offen und werden auf dieser Seite vom Gehäuse begrenzt, wobei sie eine Freilaufseite, die so groß ist, dass eine Klemmrolle nicht mit dem Gehäuse in Kontakt kommt, und eine Sperrseite aufweisen, die so klein ist, dass eine Klemmrolle mit dem Gehäuse in Kontakt kommt.

Mit Hilfe der beiliegenden Zeichnungen wird nachfolgend ein Ausführungsbeispiel der vorliegenden Erfindung näher erläutert. Es zeigen:
- Figur 1 -: die schematische Darstellung eines Teils einer orthopädietechnischen Einrichtung gemäß einem ersten Ausführungsbeispiel der vorliegenden Erfindung,
- Figur 2 -: die Darstellung aus Figur 1 mit dem Überlastschutz in einer Explosionsdarstellung,
- Figur 3 -: ein Teil des Überlastschutzes aus Figur 2,
- Figur 4 -: eine Schnittdarstellung durch die in Figur 3 gezeigten Bauteile,
- Figur 5 -: einen vergrößerten Ausschnitt aus Figur 4,
- Figur 6 -: eine axiale Draufsicht auf die in Figur 3 gezeigten Bauteile,
- Figur 7 -: einen vergrößerten Ausschnitt aus Figur 6 und
- Figur 8 -: eine Explosionsdarstellung eines Gesperres

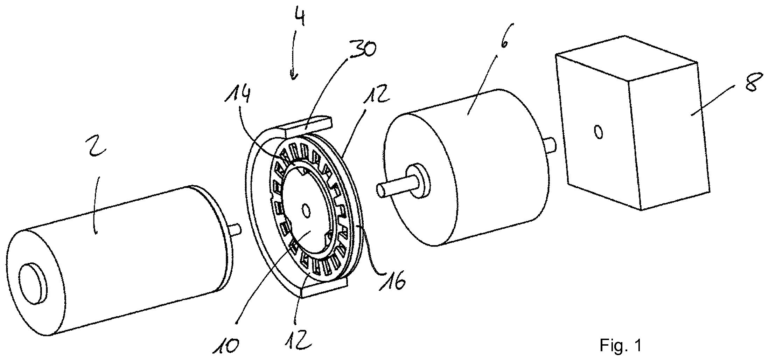

Figur 1 zeigt eine Antriebseinrichtung 2, die beispielsweise als Elektromotor ausgebildet sein kann. Sie ist über einen Überlastschutz 4 und ein Getriebe 6 mit einem Endeffektor 8 gekoppelt, der lediglich schematisch dargestellt ist.

Der Überlastschutz 4 verfügt über eine Sperreinrichtung 10, die innerhalb erster Kopplungselemente 12, zwischen denen ein zweites Kopplungselement 14 angeordnet ist und die über einen Rückschlussring 16 miteinander verbunden sind, angeordnet. Der Rückschlussring 16 sowie die ersten Kopplungselemente 12 und das zweite Kopplungselement 14 sind in einem Gehäuse 30 angeordnet.

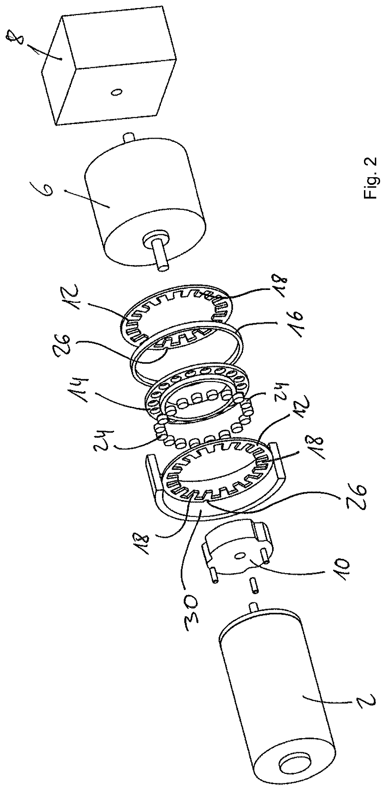

Figur 2 zeigt die Darstellung aus Figur 1 mit der Antriebseinrichtung 2, dem Getriebe 6 und dem Endeffektor 8, wobei der Überlastschutz 4 in einer Explosionsdarstellung dargestellt ist.

Man erkennt die Sperreinrichtung 10 und verschiedene Ringe, die ineinander und nebeneinander angeordnet sind. Die ersten Kopplungselemente 12 sind als ringförmige Elemente ausgebildet, die über eine Mehrzahl von nach innen ragenden Laschen 18 verfügt, die als Kopplungsbereiche fungieren. Zwischen den beiden ersten Kopplungselementen 12 befindet sich ein zweites Kopplungselement 14, das mit einer Innenseite 20 an der Sperreinrichtung 10 anliegt und über eine Mehrzahl von Ausnehmungen 22 verfügt, in die jeweils ein Magnet 24 eingesetzt ist. Das zweite Kopplungselement 14 wird umgeben durch den Rückschlussring 16, der im montierten Zustand drehfest mit den ersten Kopplungselementen 12 verbunden ist. Die ersten Kopplungselemente 12 sowie der Rückschlussring 16 verfügen über jeweils eine kleine Nut 26 durch die sie drehfest in einem Gehäuse angeordnet werden können.

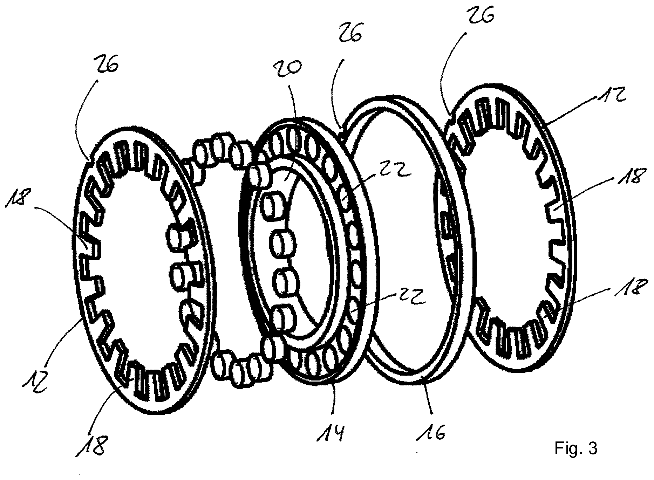

Figur 3 zeigt einige Teile des Überlastschutzes 4 in der Explosionsdarstellung vergrößert. Man erkennt insbesondere nun die Nut 26 in den ersten Kopplungselementen 12 und dem Rückschlussring 16. Man erkennt zudem, dass die Innenseite 20 des zweiten Kopplungselementes 14 in axialer Richtung, in Figur 3 also von links nach rechts, breiter ist als der Teil des zweiten Kopplungselementes 14 in dem sich die Ausnehmungen 22 befinden. Dieser Teil des zweiten Kopplungselementes 14 dient zudem als radiale Lagerung für die beiden ersten Kopplungselemente 12, die mit ihren Laschen 18 an diesem Teil des zweiten Kopplungselementes 14 anliegen.

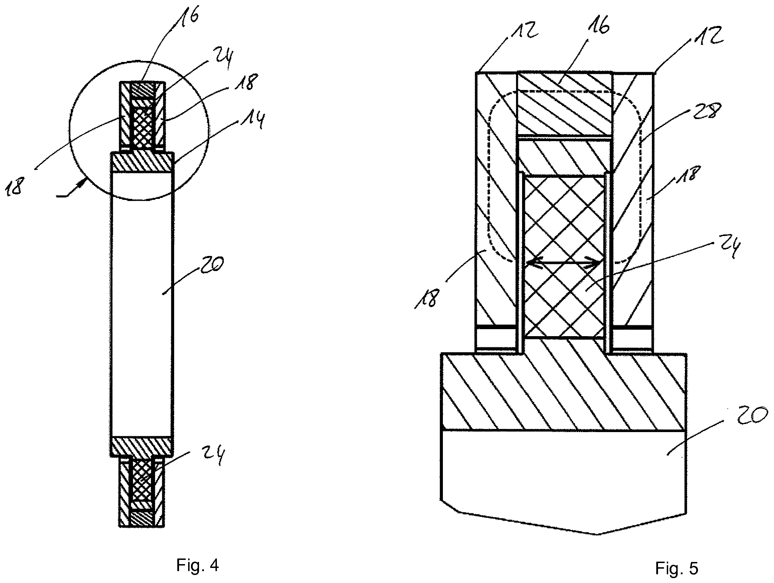

Figur 4 zeigt eine Schnittdarstellung durch die in Figur 3 gezeigten Bauteile. Die Innenseite 20 des zweiten Kopplungselementes 14 ist breiter als der restliche Teil des zweiten Kopplungselementes 14. Die Magnete 24 werden von den jeweiligen Laschen 18 und dem Rückschlussring 16 umgeben. Dies ist in Figur 5 deutlicher zu erkennen, die einen Ausschnitt aus Figur 4 zeigt. Man erkennt einen der Magneten 24, der in Figur 5 rechts und links von jeweils einer Lasche 18 eines der beiden ersten Kopplungselemente 12 abgedeckt werden. Radial außen sind die beiden ersten Kopplungselemente 12 durch den Rückschlussring 16 miteinander verbunden. Da zumindest die Laschen 18, vorteilhafterweise jedoch die gesamten ersten Kopplungselemente 12 und besonders vorteilhafterweise auch der Rückschlussring 16 aus einem magnetisierbaren Material bestehen, können die vom Magneten 24 erzeugten Feldlinien in dem schematisch dargestellten ringförmigen Verlauf 28 geführt werden.

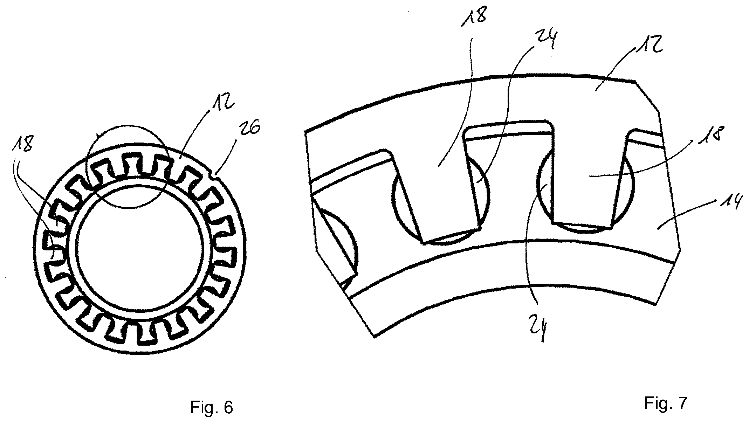

Figur 6 zeigt eine schematische Draufsicht auf eines der ersten Kopplungselemente 12 mit der Nut 26 und den nach innen ragenden Laschen 18. Figur 7 zeigt einen vergrößerten Ausschnitt, bei dem ein Teil des ersten Kopplungselementes 12 mit zwei Laschen 18 sowie den darunter liegenden Magneten 24 im zweiten Kopplungselement 14 zu erkennen sind. Wird durch die Sperreinrichtung 10 ein auf den Endeffektor 8 wirkendes Drehmoment auf das zweite Kopplungselement 14 übertragen, ist zunächst eine Bewegung des zweiten Kopplungselementes 14 relativ zum ersten Kopplungselement 12 durch die Kräfte verhindert, die durch die Magneten 24 und die Laschen 18 der ersten Kopplungselemente 12 hervorgerufen werden. Erst wenn ein dadurch definiertes Grenzdrehmoment überschritten wird, kann das zweite Kopplungselement 14 relativ zu den ersten Kopplungselementen 12 bewegt werden. Dazu müssen die Magneten 24 relativ zu den Laschen 18 verschoben werden, wofür ein Drehmoment benötigt wird. Nach einer Verschiebung, die dem Abstand zwischen zwei Laschen 18 entspricht, der vorzugsweise auch im Abstand zwischen den Ausnehmungen 22, in denen sich die Magneten 24 befinden, entspricht, ist die Situation, wie sie in Figur 7 vergrößert dargestellt ist, wieder erreicht und erneut muss das Grenzdrehmoment überwunden werden.

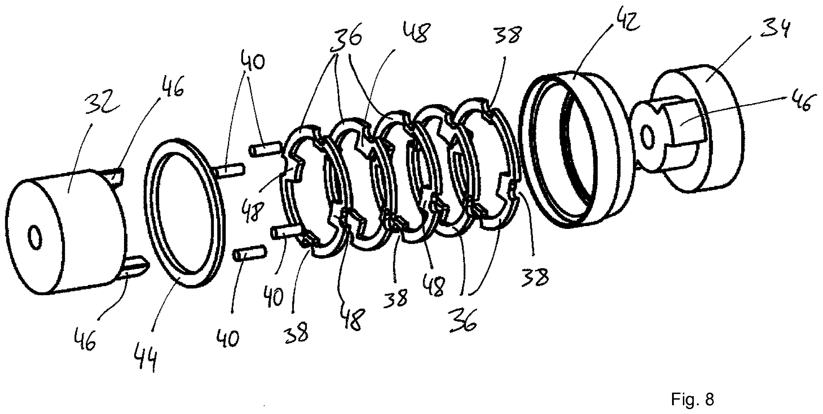

Figur 8 zeigt eine Explosionsdarstellung eines Klemmrollengesperres mit einem antriebsseitigen Mitnehmer 32 und einem abtriebsseitigen Mitnehmer 34. Das Klemmrollengesperre verfügt über fünf Klemmscheiben 36, die jeweils vier Ausnehmungen 38 aufweisen. Darin befinden sich vier Klemmrollen 40. Die Klemmscheiben 36 mit den Klemmrollen 40 befinden sich in einem Klemmrollengehäuse 42, das die Ausnehmungen 38 nach radial außen begrenzt. Eine Deckscheibe 44 verhindert im gezeigten Ausführungsbeispiel, dass die Klemmrollen 40 in axialer Richtung aus den Ausnehmungen 38 herauswandern können.

Sowohl der antriebsseitige Mitnehmer 32 als auch der abtriebsseitige Mitnehmer 34 verfügen über Mitnehmerklauen 46, mit denen sie mit den Antriebsvorsprüngen oder den Abtriebsvorsprüngen in Eingriff stehen oder in Eingriff gebracht werden können.

Man erkennt in Figur 8, dass die Klemmscheiben 36 versetzt zueinander angeordnet sind, sodass ein Winkel zwischen den Vorsprüngen 48 zweier benachbarter Klemmscheiben 36 im gezeigten Ausführungsbeispiel 90° beträgt.

### Bezugszeichenliste

- 2: Antriebseinrichtung
- 4: Überlastungsschutz
- 6: Getriebe
- 8: Endeffektor
- 10: Sperreinrichtung
- 12: erstes Kopplungselement
- 14: zweites Kopplungselement
- 16: Rückschlussring
- 18: Laschen
- 20: Innenseite
- 22: Ausnehmungen
- 24: Magnet
- 26: Nut
- 28: ringförmiger Verlauf
- 30: Gehäuse
- 32: antriebsseitiger Mitnehmer
- 34: abtriebsseitiger Mitnehmer
- 36: Klemmscheibe
- 38: Ausnehmung
- 40: Klemmrolle
- 42: Klemmrollengehäuse
- 44: Deckscheibe
- 46: Mitnehmerklaue
- 48: Vorsprung

## Patentansprüche

1. Orthopädietechnische Einrichtung mit
einem Endeffektor (8),
einer Antriebseinrichtung (2), die eingerichtet ist, den Endeffektor (8) durch Übertragen eines Drehmomentes von der Antriebseinrichtung (2) auf den Endeffektor (8) anzutreiben, und
einem Überlastungsschutz (4),
**dadurch gekennzeichnet, dass** der Überlastschutz (4) eingerichtet ist, eine Übertragung eines Drehmomentes von dem Endeffektor (8) auf die Antriebseinrichtung (2) nur bis zu einem Grenzdrehmoment in zumindest einer Richtung zu verhindern und oberhalb des Grenzdrehmoments zu gestatten, wobei der Überlastschutz (4) wenigstens ein erstes Kopplungselement (12) und ein zweites Kopplungselement (14) aufweist, die relativ zueinander drehbar und magnetisch gekoppelt sind.

2. Orthopädietechnische Einrichtung nach Anspruch 1, **dadurch gekennzeichnet, dass** das zweite Kopplungselement (14) eine Mehrzahl von Magneten (24), vorzugsweise Permanentmagneten, aufweist.

3. Orthopädietechnische Einrichtung nach Anspruch 2, **dadurch gekennzeichnet, dass** die Magneten (24) lösbar in dafür an dem zweiten Kopplungselement (14) vorhandenen Vertiefungen und/oder Ausnehmungen (22) angeordnet sind.

4. Orthopädietechnische Einrichtung nach Anspruch 3, **dadurch gekennzeichnet, dass** die Vertiefungen und/oder Ausnehmungen (22) äquidistant über zumindest einen Abschnitt des Umfanges, vorzugsweise den vollständigen Umfang des zweiten Kopplungselementes (14) angeordnet sind.

5. Orthopädietechnische Einrichtung nach einem der Ansprüche 2 bis 4, **dadurch gekennzeichnet, dass** die Einrichtung zwei erste Kopplungselemente (12) aufweist, zwischen denen das zweite Kopplungselement (14) angeordnet ist.

6. Orthopädietechnische Einrichtung nach einem der Ansprüche 2 bis 5, **dadurch gekennzeichnet, dass** wenigstens eines der ersten Kopplungselemente (12) eine Mehrzahl von Kopplungsbereichen, insbesondere in Form von radial nach innen ragender Laschen (18), aufweist, wobei vorzugsweise eine Anzahl der Laschen (18) der Anzahl der Magnete (24) und/oder einer Anzahl der Vertiefungen und/oder Ausnehmungen (22) entspricht.

7. Orthopädietechnische Einrichtung nach einem der vorstehenden Ansprüche, **dadurch gekennzeichnet, dass** das wenigstens eine erste Kopplungselement (12) oder das zweite Kopplungselement (14) drehfest an einem Gehäuse angeordnet sind.

8. Orthopädietechnische Einrichtung nach einem der vorstehenden Ansprüche, **dadurch gekennzeichnet, dass** der Überlastschutz (14) eine Sperreinrichtung (10) aufweist, die eingerichtet ist, die Übertragung des Drehmomentes von der Antriebseinrichtung (2) auf den Endeffektor (8) nicht zu beeinflussen und die Übertragung des Drehmomentes von dem Endeffektor (8) auf die Antriebseinrichtung (2) bis zu dem Grenzdrehmoment zu verhindern.

9. Orthopädietechnische Einrichtung nach Anspruch 8, **dadurch gekennzeichnet, dass** die Sperreinrichtung (10) ein Klemmrollengesperre mit einem Gehäuse (42) und
mindestens zwei, vorzugsweise mindestens drei Klemmscheiben (36) aufweist, die
• in dem Gehäuse (42) drehbar angeordnet sind und
• jeweils wenigstens eine Ausnehmung (38), in der sich eine Klemmrolle (40) befindet,
• wenigstens einen Antriebsvorsprung und
• wenigstens einen Abtriebsvorsprung aufweisen,
wobei die Klemmscheiben (36) derart angeordnet und ausgebildet sind, dass ein Drehen der Klemmscheiben (36) durch Antreiben der Antriebsvorsprünge (12) möglich ist und beim Antreiben der Abtriebsvorsprünge ein Drehen der Klemmscheiben (36) verhindert wird.

10. Orthopädietechnische Einrichtung nach Anspruch 9, **dadurch gekennzeichnet, dass** die Ausnehmungen (38) der Klemmscheiben (36) nach radial außen offen sind und auf dieser Seite vom Gehäuse (42) begrenzt werden, wobei sie eine Freilaufseite, die so groß ist, dass eine Klemmrolle (40) nicht mit dem Gehäuse (42) in Kontakt kommt, und eine Sperrseite aufweisen, die so klein ist, dass eine Klemmrolle (40) mit dem Gehäuse (42) in Kontakt kommt.

## Claims

1. An orthopaedic device with
an end effector (8),
a driving device (2) which is configured to drive the end effector (8) by transmitting a torque from the driving device (2) to the end effector (8), and
an overload protection (4),
**characterized by the fact that** the overload protection (4) is configured to only prevent a transmission of a torque from the end effector (8) to the driving device (2) up to a threshold torque in at least one direction and to allow it above the threshold torque, wherein the overload protection (4) comprises at least one first coupling element (12) and a second coupling element (14), which can be rotated relative to one another and which are coupled magnetically.

2. The orthopedic device according to claim 1, **characterized by the fact that** the second coupling element (14) comprises a plurality of magnets (24), preferably permanent magnets.

3. The orthopedic device according to claim 2, **characterized by the fact that** the magnets (24) are detachably arranged in specially provided indentations and/or recesses (22) on the second coupling element (14).

4. The orthopedic device according to claim 3, **characterized by the fact that** the indentations and/or recesses (22) are arranged to be equidistant across at least one section of the circumference, preferably across the entire circumference, of the second coupling element (14).

5. The orthopedic device according to one of the claims 2 to 4, **characterized by the fact that** the device has two first coupling elements (12), between which the second coupling element (14) is arranged.

6. The orthopedic device according to one of the claims 2 to 5, **characterized by the fact that** at least one of the first coupling elements (12) has a plurality of coupling regions, which are designed - in particular - in the form of tabs (18) that protrude radially inwards, wherein preferably a number of the tabs (18) corresponds to the number of the magnets (24) and/or a number of the indentations and/or recesses (22).

7. The orthopedic device according to one of the above claims, **characterized by the fact that** at least one first coupling element (12) or the second coupling element (14) is arranged on a housing in a torque-proof way.

8. The orthopedic device according to one of the above claims, **characterized by the fact that** the overload protection (14) has a locking device (10) that is configured to not effect the transfer of the torque from the driving device (2) to the end effector (8) and to prevent the transfer of the torque from the end effector (8) to the driving device (2) up until the point of the threshold torque.

9. The orthopedic device according to claim 8, **characterized by the fact that** the locking device (10) comprises a jamming roller lock with
a housing (42) and
at least two, preferably at least three, clamping discs (36) which
• are arranged in the housing (42) such that they can be rotated and
• comprise at least one recess (38), in which a jamming roller (40) is situated,
• at least one driving projection and
• at least one driven projection,
wherein the clamping discs (36) are arranged and designed in such a way that a rotation of the clamping discs (36) is possible by driving the driving projections (12) and a rotation of the clamping discs (36) by driving the driven projections is prevented.

10. The orthopedic device according to claim 9, **characterized by the fact that** the recesses (38) of the clamping discs (36) are open radially outwards and are restricted by the housing (42) on this side, wherein they comprise a freewheel side, which is large enough to ensure that a jamming roller (40) does not come into contact with the housing (42), and a locking side, which is small enough to ensure that a jamming roller (40) comes into contact with the housing (42).

## Revendications

1. Dispositif orthopédique comprenant
un effecteur terminal (8),
un dispositif d'entraînement (2) conçu pour entraîner l'effecteur terminal (8) par transmission d'un couple de rotation du dispositif d'entraînement (2) à l'effecteur terminal (8), et
une protection contre les surcharges (4),
**caractérisé en ce que** la protection contre les surcharges (4) est conçue pour empêcher dans au moins une direction la transmission d'un couple de rotation de l'effecteur terminal (8) au dispositif d'entraînement (2) uniquement jusqu'à un couple de rotation limite et pour la permettre au-dessus du couple de rotation limite, la protection contre les surcharges (4) comportant au moins un premier élément de couplage (12) et un deuxième élément de couplage (14) qui peuvent tourner l'un par rapport à l'autre et qui sont couplés magnétiquement.

2. Dispositif orthopédique selon la revendication 1,
**caractérisé en ce que** le deuxième élément de couplage (14) comporte une pluralité d'aimants (24), de préférence des aimants permanents.

3. Dispositif orthopédique selon la revendication 2,
**caractérisé en ce que** les aimants (24) sont disposés de manière amovible dans des renfoncements et/ou des évidements (22) prévus à cet effet sur le deuxième élément de couplage (14).

4. Dispositif orthopédique selon la revendication 3,
**caractérisé en ce que** les renfoncements et/ou les évidements (22) sont disposés de manière équidistante sur au moins une partie de la périphérie, de préférence sur toute la périphérie du deuxième élément de couplage (14).

5. Dispositif orthopédique selon l'une des revendications 2 à 4,
**caractérisé en ce que** le dispositif comprend deux premiers éléments de couplage (12) entre lesquels est disposé le deuxième élément de couplage (14).

6. Dispositif orthopédique selon l'une des revendications 2 à 5,
**caractérisé en ce qu'**au moins l'un des premiers éléments de couplage (12) présente une pluralité de zones de couplage, en particulier sous forme de pattes (18) faisant saillie radialement vers l'intérieur, et, de préférence, un nombre de pattes (18) correspond au nombre d'aimants (24) et/ou à un nombre de renfoncements et/ou d'évidements (22).

7. Dispositif orthopédique selon l'une des revendications précédentes, **caractérisé en ce que** ledit au moins un premier élément de couplage (12) ou le deuxième élément de couplage (14) est disposé solidairement en rotation sur un boîtier.

8. Dispositif orthopédique selon l'une des revendications précédentes, **caractérisé en ce que** la protection contre les surcharges (14) comporte un dispositif de blocage (10) qui est conçu pour ne pas influencer la transmission du couple de rotation du dispositif d'entraînement (2) à l'effecteur terminal (8) et pour empêcher la transmission du couple de rotation de l'effecteur terminal (8) au dispositif d'entraînement (2) jusqu'au couple de rotation limite.

9. Dispositif orthopédique selon la revendication 8,
**caractérisé en ce que** le dispositif de blocage (10) est un mécanisme de blocage à galets de serrage comportant
un boîtier (22) et
au moins deux, de préférence au moins trois disques de serrage (2) qui
• sont disposés de manière rotative dans le boîtier (22) et
• présentent chacun au moins un évidement (38) dans lequel se trouve un galet de serrage (40),
• au moins une saillie menante (12) et
• au moins une saillie menée (14),
les disques de serrage (36) étant disposés et conçus de telle sorte qu'une rotation des disques de serrage (36) est possible par entraînement des saillies menantes (12) et qu'une rotation des disques de serrage (2) est empêchée lors de l'entraînement des saillies menées (14).

10. Dispositif orthopédique selon la revendication 9,
**caractérisé en ce que** les évidements (4) des disques de serrage (2) sont ouverts radialement vers l'extérieur et sont délimités de ce côté par le boîtier (42), en présentant un côté de roue libre qui est suffisamment grand pour qu'un galet de serrage (40) ne vienne pas en contact avec le boîtier (42), et un côté de blocage qui est suffisamment petit pour qu'un galet de serrage (40) vienne en contact avec le boîtier (42).
